Europäisches Patentamt

European Patent Office

Office européen des brevets

(19)

(11) Publication number: **0 052 898**
**B1**

(12) **EUROPEAN PATENT SPECIFICATION**

(45) Date of publication of patent specification: **02.05.84**

(21) Application number: **81201212.8**

(22) Date of filing: **29.10.81**

(51) Int. Cl.³: **C 07 D 213/08,**
**C 07 D 215/06,**
**C 07 D 211/12,**
**C 07 D 211/02**

(54) **Process for the preparation of C-substituted pyridines and/or hydrogenated C-substituted pyridines.**

(30) Priority: **15.11.80 NL 8006256**

(43) Date of publication of application:
**02.06.82 Bulletin 82/22**

(45) Publication of the grant of the patent:
**02.05.84 Bulletin 84/18**

(84) Designated Contracting States:
**BE CH DE FR GB IT LI NL SE**

(56) References cited:
**EP - A - 0 009 289**
**US - A - 3 695 841**

(73) Proprietor: **STAMICARBON B.V.**
**Postbus 10**
**NL-6160 MC Geleen (NL)**

(72) Inventor: **De Graaf, Theodorus Franciscus Maria**
**Reinierstraat 2**
**NL-6191 SH Beek (NL)**
Inventor: **Geersheuvels, Charles Hendricus**
**Van Ostadestraat 76**
**NL-6165 XM Geleen (NL)**

(74) Representative: **Pinckaers, August René et al,**
**OCTROOIBUREAU DSM Postbus 9**
**NL-6160 MA Geleen (NL)**

The file contains technical information
submitted after the application was filed and not
included in this specification

Courier Press, Leamington Spa, England.

**0 052 898**

Process for the preparation of c-substituted pyridines and/or hydrogenated c-substituted pyridines

The invention relates to a process for the preparation of pyridines and/or hydrogenated pyridines substituted to one or more C-atoms with a hydrocarbon group by catalytic cyclisation, in the gas phase, of a $\gamma$-cyanoketone in the presence of hydrogen.

In applying this process known in the art (see British patent specifications 1,304,155 and 1,425,698) a gaseous reaction mixture is obtained containing a fairly large quantity of hydrogen. After separation from the reaction mixture, this hydrogen can be used again. In order to keep the activity and selectivity of the catalyst used in the cyanoketone cyclisation process at a high level in such a hydrogen recirculation process, it has already been suggested (see European patent application 9289) to pass the hydrogen-containing gas to be recirculated over a catalyst containing iron, nickel, cobalt or a compound of one of these metals at a temperature of 300—800°C.

It has now been found, however, that in such a catalytic treatment of the hydrogen-containing gas to be recirculated there will be a deposit of carbon on the catalyst and in the equipment used in this process in such a quantity that after some time, for instance after 6 days, the activity of the catalyst will be adversely affected and the equipment will cease to function as required. The recirculation of the hydrogen-containing gas must then be interrupted for the purpose of replacing the catalyst and cleaning the equipment, which is a rather costly affair.

It has now been found that in the catalytic treatment of the hydrogen-containing gas to be recirculated, the deposit of carbon can be avoided, at least be reduced to an acceptable minimum, by carrying out this treatment in two steps.

The process according to the invention for the preparation of pyridines and/or hydrogenated pyridines substituted on one or more C-atoms with a hydrocarbon group by catalytic cyclisation, in the gas phase, of a $\gamma$-cyanoketone in the presence of hydrogen, separating hydrogen-containing gas from the reaction mixture obtained and recirculating it after catalytic treatment is charcterized in that the catalytic treatment of the hydrogen-containing gas to be recirculated is carried out in two steps and the gas to be recirculated is passed, in the first step, at a temperature of 300—500°C, over a nickel-containing catalyst and, in the second step, at a temperature of 575—700°C, over a nickel-containing catalyst.

For the catalytic treatment of the hydrogen-containing gas to be recirculated according to the invention, the nickel-containing catalysts known in themselves can be used. These catalysts may contain the metal as such and/or a compound of the metal, for instance an oxide of the metal. These catalysts can, furthermore, be applied on a carrier, such a aluminium oxide, silicon oxide, magnesium oxide and carbon.

In each step of the catalytic treatment of the gas to be recirculated, the space velocity at which the relative gas is passed over the catalyst can be varied within wide limits, for instance from 0.1—50 litres at N.T.P. per gramme catalyst (including any carrier applied) per hour. Preferably in both steps a space velocity is applied of between 0.25 and 20 litres at N.T.P. of hydrogen-containing gas per gramme catalyst per hour.

In applying the process according to the invention part of the hydrogen-containing gas may have to be discharged to avoid too high a concentration of impurities. In such a case, however, fresh hydrogen must be supplied to the cyanoketone to be converted for the purpose of maintaining the desired hydrogen/cyanoketone ratio. It has now been found that impure hydrogen, (containing e.g. carbon monoxide and/or ammonia) for instance hydrogen obtained as off-gas in dehydrogenations or in cracking of petroleum fractions, can then also be supplied to the gas to be recirculated, before subjecting the latter to the catalytic treatment according to the invention, to maintain the desired hydrogen/cyanoketone ratio.

In applying the process according to the invention, as in applying the process known in the art, various $\gamma$-cyanoketones can be used as starting compounds, such as 5-oxohexanenitrile, 5-oxoheptanenitrile, 4-methyl-5-oxohexanenitrile, 2-($\beta$-cyanoethyl)-cyclohexanone, 4-phenyl-5-oxohexanenitrile and 4-methyl-5-oxoheptanenitrile. The process according to the invention is very suitable for the preparation of 2-methyl-pyridine, 2,3-lutidine and quinoline starting from, respectively, 5-oxohexanenitrile, 4-methyl-5-oxohexanenitrile and 2-($\beta$-cyanoethyl)-cyclohexanone.

The compounds obtained in applying the process according to the invention can be used for various purposes, for instance for the preparation of herbicides.

In the following examples the invention will be further elucidated.

Examples I—V

Through a tubular reactor of a diameter of 25 millimetres and a length of 500 millimetres, provided with a catalyst bed (50 grammes of catalyst consisting of palladium on aluminium oxide with 0.5% by weight of Pd) and a heating jacket, a gaseous mixture of 5-oxohexanenitrile and hydrogen, obtained by evaporation of liquid 5-oxohexanenitrile and mixing of the vapour obtained thereby with hydrogen, is passed over the catalyst bed. The temperature of the catalyst bed was kept at 240°C. The space velocity of the nitrile was 0.15 gramme per gramme catalyst per hour and of the hydrogen 0.15 litre at N.T.P. hydrogen per gramme catalyst per hour.

2

The reaction mixture obtained was passed through an ice-cooled receiving tank in which the reaction product condensed. The non-condensed gaseous reaction mixture, mainly consisting of hydrogen, was passed, at elevated temperature (varied between 300 and 500°C), through a second tubular reactor (diameter 10 millimetres, length 250 millimetres) containing a bed of 7.5 grammes nickel catalyst (16.1% by weight of Ni in respect of $SiO_2$ grade G1—22 of BASF) and subsequently, at a temperature of 650°C through a third tubular reactor of the same dimensions as the second tubular reactor and filled with a bed of again 7.5 grammes of the said nickel catalyst. The gas mixture obtained from the third reactor was recirculated to the inlet of the first reactor in which the 5-oxohexanenitrile was converted. By discharging a portion of the gas to be recirculated, the concentration of hydrogen in this gas was kept above 99%. By supply of fresh hydrogen (purity 99.0%) the amount of hydrogen to be pased through was maintained at the desired value. The space velocity in the second reactor was varied between 0.35 and 6 litres at N.T.P. of hydrogen-containing gas per gramme nickel catalyst per hour. After an operating period of 10 days the quantity of 5-oxohexanenitrile passed though and the quantity of reaction product obtained were measured under constant conditions for 1 hour. The quantity of 5-oxohexanenitrile passed through was determined by measuring the loss of weight of liquid 5-oxohexanenitrile.

The reaction product collected was subjected to a gaschromatographic analysis. The results of the determinations are summarized in the following table. This table also mentions the results of a comparative example A relating to an experiment in which no hydrogen was recirculated to the first reactor.

| example | temperature nickel catalyst in 2nd reactor in °C | space velocity 2nd reactor | conversion of nitrile in % | selectivity 2-methyl-pyridine in % | selectivity 2-methyl-piperidine in % |
|---------|---------|---------|---------|---------|---------|
| I | 400 | 2 | 99.9 | 83.8 | 4.6 |
| II | 350 | 1 | 100 | 84.1 | 4.5 |
| III | 300 | 0.35 | 99.9 | 83.7 | 4.6 |
| IV | 500 | 3 | 99.8 | 84.0 | 4.5 |
| V | 450 | 6 | 99.9 | 83.9 | 4.6 |
| A | — | — | 100 | 84.2 | 4.6 |

A comparative example B without application of the second reactor has to be discontinued after 6 days, because clogging occured in the third reactor in consequence of a carbon deposit.

Example VI

Example I and the comparative examples were repeated under the same conditions but at a temperature, in the first reactor, of 230°C and with 4-methyl-5-oxohexanenitrile instead of 5-oxohexanenitrile. The conversion was 99.2%; the selectivity to 2,3-dimethylpyridine 89.1% and to 2,3-dimethylpiperidine 9.1%. With comparative example A without recirculation of hydrogen these values were 99.6%, 89.6% and 8.8% respectively, and with comparative example B, in which the hydrogen to be recirculated was fed direct, without treatment, to the third reactor 97.2%, 84.7% and 9.0% respectively. Moreover, with comparative example B the pressure differential over the third reactor showed a strong increase.

Example VII

In the same as in example I 2-(β-cyanoethyl)cyclohexanone was passed over 50 grammes of the Pd catalyst. The catalyst temperature was kept at 210°C. The space velocity of the cyanoketone was 0.15 gramme per gramme Pd catalyst per hour and of the hydrogen 0.3 litres at N.T.P. per gramme catalyst per hour. The hydrogen to be recirculated was passed at 450°C through the second reactor and subsequently, at 650°C, through the third reactor. The space velocity in the second and third reactors were 1 litre at N.T.P. per gramme catalyst per hour. For the purpose of comparison an experiment was made without recirculation of hydrogen and, under the same conditions, an experiment with recirculation of hydrogen in which the gas mixture to be recirculated (hydrogen mainly) was passed, without treatment, direct to the third reactor. For the example and the comparative experiment without recirculation the conversion was 99.8% and 100% respectively. In both cases the selectivity to quinoline was 1%; the selectivity to decahydroquinoline was, in both cases, 48%; the selectivity to 1,2,3,4-tetrahydroquinoline 5% and 6% respectively and the selectivity to 5,6,7,8-tetrahydroquinoline 43% and 42% respectively. The comparative experiment with the recirculation of hydrogen only via the third reactor has to be discontinued prematurely on account of clogging.

3

# 0 052 898

## Claims

1. Process for the preparation of pyridines and/or hydrogenated pyridines substituted on one or more C-atoms with a hydrocarbon group by catalytic cyclisation, in the gas phase, of a $\gamma$-cyanoketone in the presence of hydrogen, separating hydrogen-containing gas from the reaction mixture obtained and recirculating it after catalytic treatment, characterized in that the catalytic treatment of the hydrogen-containing to be recirculated is carried out in two steps, and the gas to be recirculated is passed, in the first step, at a temperature of 300—500°C, over a nickel-containing catalyst and, in the second step, at a temperature of 575—700°C, over a nickel-containing catalyst.

2. Process according to claim 1, characterized in that in both steps a space velocity is applied of 0.25—20 litres at N.T.P. of hydrogen-containing gas per gramme catalyst per hour.

3. Process according to any one of claims 1—2, characterized in that part of the hydrogen-containing gas to be recirculated is discharged and the desired hydrogen/cyanoketone ratio is maintained by supplying impure hydrogen in addition to the gas to be recirculated before subjecting the latter to the catalytic treatment.

## Revendications

1. Procédé pour la préparation de pyridines et/ou de pyridines hydrogénées substituées sur un ou plusieurs atomes de carbone par un groupe d'hydrocarbure par cyclisation catalytique, dans la phase gazeuse, d'une gammacyanocétone en présence d'hydrogène, séparation d'un gaz contenant de l'hydrogène de mélange de réaction obtenu et recyclage de ce gaz après traitement catalytique, caractérisé en ce que le traitement catalytique du gaz contenant de l'hydrogène à recycler est effectué en deux étapes et que le gaz à recycler est passé, dans la première étape, à une température de 300—500°C, sur un catalyseur contenant du nickel et, dans la seconde étape, à une température de 575—700°C, sur un catalyseur contenant du nickel.

2. Procédé selon la revendication 1, caractérisé en ce que dans les deux étapes on utilise une vitesse spatiale de 0,25—20 litres (à température et pression normales) de gaz contenant de l'hydrogène par gramme de catalyseur et par heure.

3. Procédé selon l'une quelconque des revendications 1—2, carctérisé en ce qu'une partie du gaz contenant de l'hydrogène à recycler est déchargée et que l'on maintient le rapport hydrogène/cyano-cétone désiré en ajoutant de l'hydrogène impur au gaz à recycler avant de soumettre ce dernier au traitement catalytique.

## Patentansprüche

1. Verfahren zur Herstellung von Pyridinen und/oder hydrierten Pyridinen, substituiert an einem oder mehreren C-Atomen mit einer Kohlenwasserstoffgruppe durch katalytische Ringbildung in der Gasphase eines $\gamma$-Cyanketons in Anwesenheit von Wasserstoff, Trennung des wasserstoffhaltigen Gases vom erhaltenen Reaktionsgemisch und Rückführung dieses Gases nach katalytischer Behandlung, dadurch gekennzeichnet, dass die katalytische Behandlung des zurückzuführenden wasserstoffhaltigen Gases in zwei Schritten erfolgt und das zurückzuführende Gas in ersten Schritt bei einer Temperatur von 300—500°C über einen Nickel enthaltenden Katalysator geführt wird und im zweiten Schritt bei einer Temperatur von 575—700°C über einen Nickel enthaltenden Katalysator geführt wird.

2. Verfahren nach Anspruch 1, dadurch gekennzeichnet, dass in beiden Schritten eine Raumgeschwindigkeit von 0,25—20 Litern (Normaltemperatur und -druck) wasserstoffhaltigem Gas je Gramm Katalysator je Stunde angewandt wird.

3. Verfahren nach einem der Ansprüche 1—2, dadurch gekennzeichnet, dass ein Teil des zurückzuführenden wasserstoffhaltigen Gases abgeführt wird und das erwünschte Wasserstoff/Cyanketon-Verhältnis aufrechterhalten wird indem unreiner Wasserstoff dem zurückzuführenden Gas zugesetzt wird bevor dieses der katalytischen Behandlung unterworfen wird.

4